# EUROPEAN PATENT SPECIFICATION

(11) **EP 3 116 399 B1**
(45) Date of publication and mention of the grant of the patent: **23.10.2019**
(21) Application number: 15760821.7
(22) Date of filing: 16.03.2015
(51) Int. Cl.: A61B 5/15, A61M 25/06, A61B 17/34, A61B 17/122, A61B 17/04, A61B 17/00

(54) **A CANNULATION ASSEMBLY**
PUNKTIONSANORDNUNG
ENSEMBLE DE CANULATION

(30) Priority: 14.03.2014 SG 10201400686V
(43) Date of publication of application: 18.01.2017
(73) Proprietor: SG MediTech Pte Ltd, Singapore 408564 (SG)
(72) Inventor: WIGHT, Ron, Singapore 408564 (SG); TAN, Theodore, Singapore 408564 (SG); TAN, Gabriel, Singapore 408564 (SG); LEE, Chun Siong, Singapore 408564 (SG)
(74) Representative: Talbot-Ponsonby, Daniel Frederick
(86) International application number: PCT/IB2015/000333
(87) International publication number: WO 2015/136361

(56) References cited:
- WO-A1-2013/112106
- WO-A1-2014/007601
- WO-A2-2008/005618
- US-A- 4 627 841
- US-A1- 2004 243 096
- US-A1- 2009 177 161
- US-B1- 8 029 471

## Description

### Field of the Invention

The invention relates to the extraction of umbilical cord blood and, in particular, an apparatus to achieve this.

### Background of Invention

It is well known that umbilical cord blood (UCB) is an increasingly important and rich source of stem cells. It is known that stem cells can divide to create new red blood cells which carry oxygen to the brain, new white blood cells used in the body's immune system and new platelets which can assist in blood clotting. It is currently estimated that stem cells may be used for the treatment of over 45 malignant and non-malignant diseases. Such diseases may include certain cancers such as leukemia, immune and genetic disorders.

UCB may also provide a readily available source of stem cells for transplantation in many situations where bone marrow is currently used. Hence, the use of UCB instead of other sources of stem cells such as for example bone marrow and peripheral blood has many advantages. Such may include for example the reduction or elimination of risk involved in the collection of UCB. UCB is also easier to collect and harvest while avoiding the risks associated with general anesthesia, which is required for the purposes of extracting bone marrow.

UCB is also readily available when needed, assuming an efficient and systematic collection and storage procedure. It has been found that UCB is also more often compatible with people undergoing transplants. Furthermore UCB has a lower procurement cost. It has also been demonstrated that UCB has broader potential clinical applications for improving neural repair, bone and tissue growth. As such, the importance of UCB is now widely recognized. Blood centres worldwide may collect and store UCB after delivery of a baby subject to the parents' consent or request. However, a problem associated with UCB is that its collection appears to be a one-time possibility and the amount of blood that can be collected is limited using current blood collection technology. Such current blood collection technology may include syringe assisted and gravity assisted methods. WO2013/112106 is one example of a device and method for extracting and/or collecting placental blood.

### Summary of Invention

The invention provides a cannulation assembly comprising: a housing; a needle selectively extendable from a proximal end of said housing; a tube arranged to pass through the housing and through a bore of said needle such that an insertion end of said tube is arranged to selectively project from an insertion end of said needle; a slide mounted to the needle in sliding engagement with the housing and arranged to move the needle from a retracted position to an insertion position such that the needle projects from said housing; wherein said slide is further arranged to move the needle from the insertion position to a locked position such that the needle is fully retracted; and wherein the insertion end is spherical in shape, said spherical insertion end arranged to form an annular seal against an inside diameter of the needle.

Also disclosed herein is a clamp for clamping an umbilical cord, the clamp comprising: two arms connected by a resilient hinge, said hinge arranged to bias said arms to an open position; one arm having a release lever at an end opposed to the hinge; the second arm having at least one ridge at an end opposed to the hinge; said lever and at least one ridge cooperatively shaped and arranged to engage on closing of said arms and, on activation of the lever, said arms are opened under the resilient biasing of said hinge; wherein said lever is arranged to activate on depressing a contact point on said lever.

Also disclosed herein is a cannulation needle comprising a bevel proximate to a tip, said bevel including a reverse curve.

Accordingly, the invention provides for the needle to be used to insert the tube, and can then be removed to allow access for the tube. Further, by passing the tube through the housing, and importantly, through the needle the volume of blood extracted is maximized through having the larger bore of the needle during cannulation.

In one embodiment, the needle may also be locked in place after retraction so as to prevent multiple uses of the cannulation assembly. In this embodiment, the first lock may therefore prevent cross contamination through use with multiple patients.

### Brief Description of Drawings

It will be convenient to further describe the present invention with respect to the accompanying drawings that illustrate possible arrangements of the invention. Other arrangements of the invention are possible and consequently, the particularity of the accompanying drawings is not to be understood as superseding the generality of the preceding description of the invention.
Figure 1 is an elevation view of a cannulation assembly according to one embodiment of the present invention;

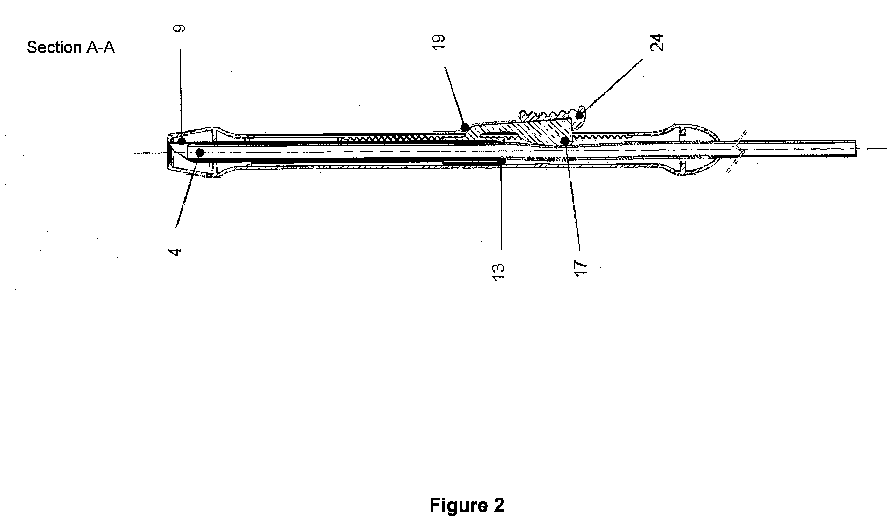

Figure 2 is a sectional view of the cannulation assembly of Figure 1;

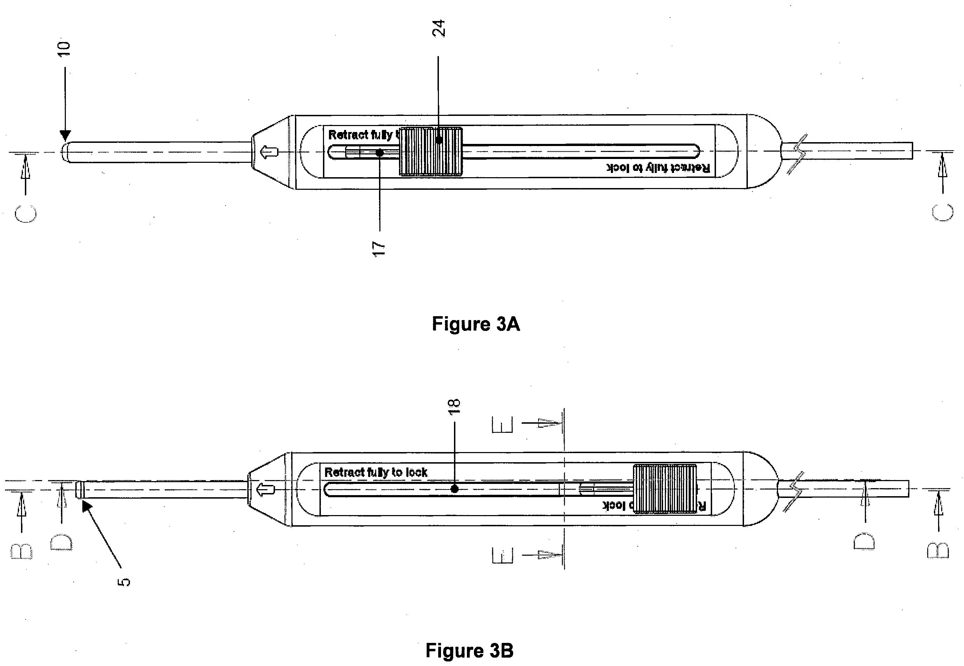

Figures 3A and 3B are plan views of the cannulation assembly according to a further embodiment of the present invention;
Figures 4 to 7 are sectional views of the cannulation assembly of Figures 3A and 3B;
Figure 8 provides various views of an umbilical cord clamp according to one embodiment of the present invention;
Figure 9 provides various sectional views of the cannulation assembly of Figure 3A;
Figures 10 and 11 are exploded views of the cannulation assembly of Figure 1.
Figures 12A, 12B, 13A and 13B are isometric views of a cannulation procedure according to one embodiment of the present invention;
Figure 14 is a schematic view of a full cannulation arrangement according to a further embodiment of the present invention;
Figures 15A to 15C are various views of a needle for a cannulation assembly according to a further embodiment of the present invention.

### Detailed Description

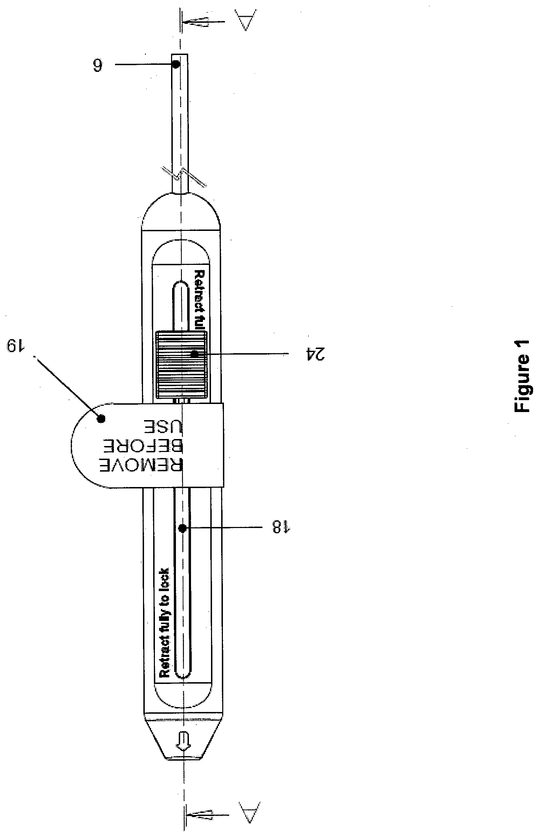

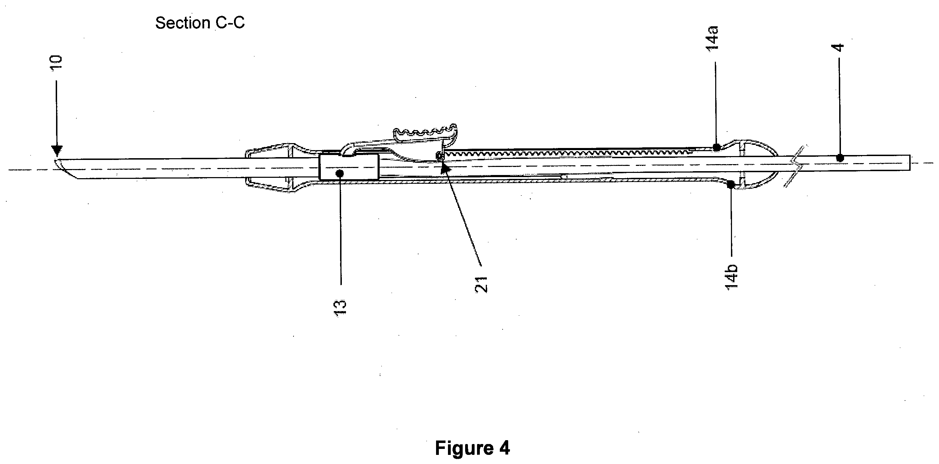

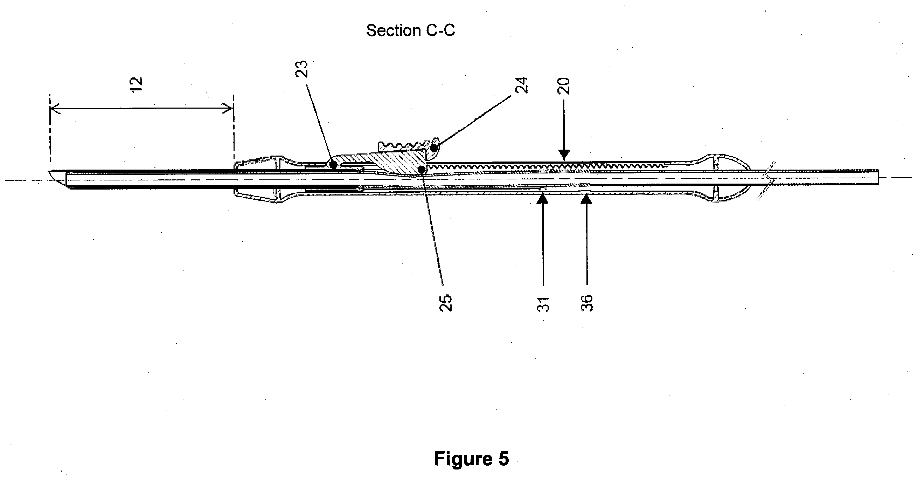

Figure 1 shows one embodiment where the cannula itself is a length of flexible plastic tubing 4 of suitable composition for the safe transfer and storage of blood. The distal end, or tube insertion end, is spherically tipped 5 whilst the proximal end 6 is connected to the tube set; providing in-utero collection of blood into a removable blood bag 7 and thereafter, redirection and ex-utero collection via a second removable blood bag 8. The cannula slides through the inside diameter of a surgical stainless steel needle 9 or trocar; the push/pull force being characteristic of a transition fit. The needle has a distal taper point, or needle insertion end, 10 (Figure 4 and 5), the proximal end 11 being square and perpendicular and bonded to the carriage tube 13. The length of the needle is sufficient to allow adequate extension 12 from the cannula housing to perforate the umbilical vein. The proximal end of the needle is inserted into the carriage tube 13 which is assembled within a two piece plastic housing, the upper 14a and the lower 14b; suitably bonded together in a manner that prevents disassembly. The carriage tube 13 is guided and restrained laterally within the lower housing 14b by a combination of ribs 15 and raised projections 16; allowing the carriage to extend and retract with minimal frictional contact. Extending posteriorly with proximal bias from the carriage tube is the carriage lever 17. A slot 18 in the upper housing allows the carriage lever to protrude. In doing so, the carriage is guided linearly and prevented from rotation.

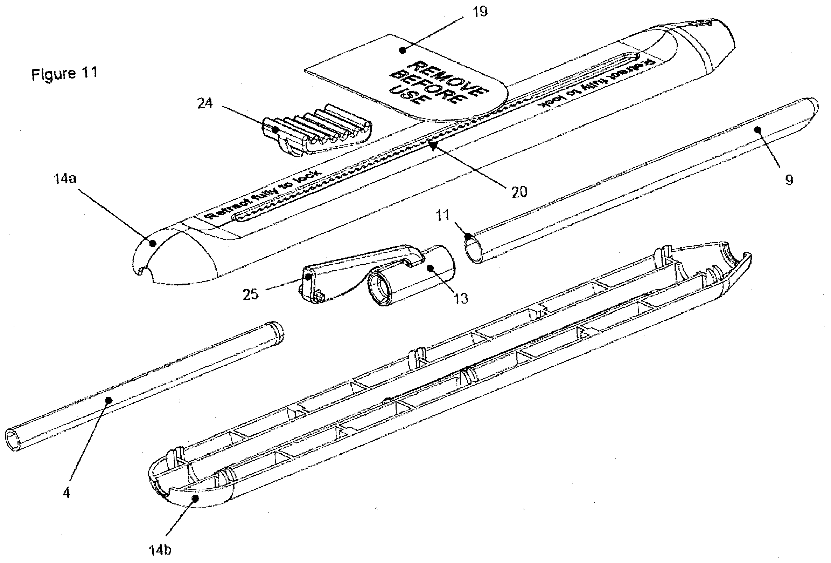

In the as packaged configuration, the spherical tip of the cannula 5 is positioned slightly proximal to the distal opening of the needle, whilst the needle is positioned within the housing; the distal taper point 10 being concealed within the housing. An external adhesive label 19, wraps over the lower to upper housing joint and the carriage lever (Figure 1, 10 and 11). The label maintains the as packaged position of the distal taper point of the needle relative to the housing. It must be peeled off and removed prior to use. Once removed, the cannula shall be considered as used and non-sterile.

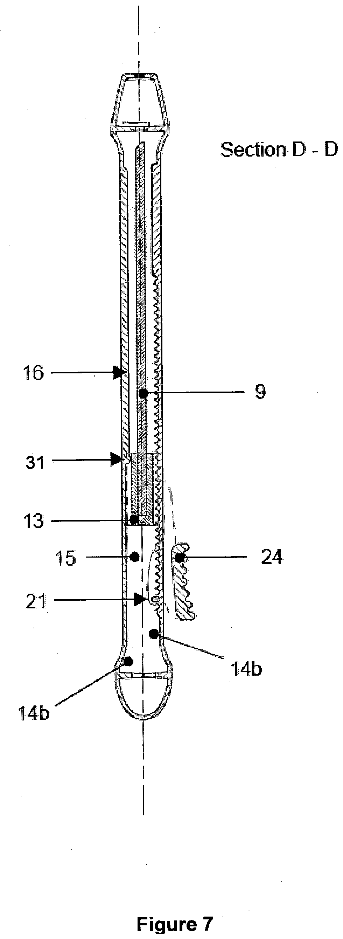

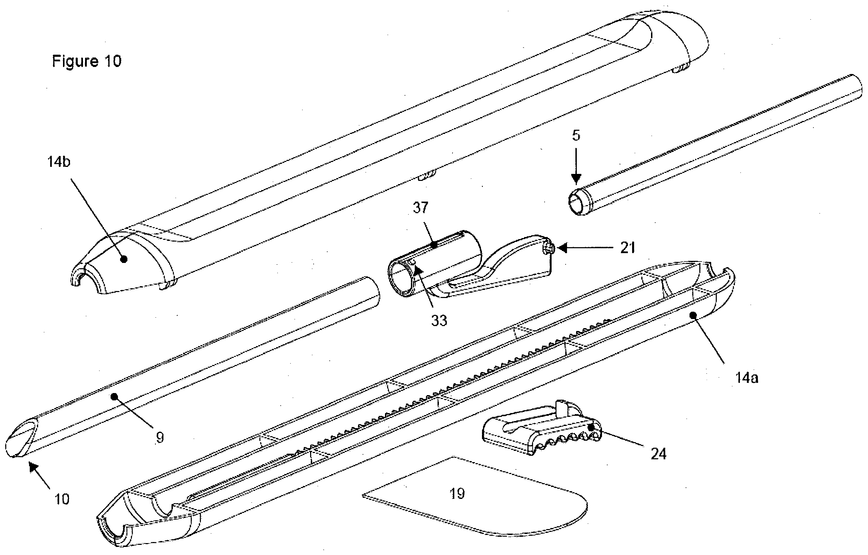

Immediately posterior to the label, on the inside of the upper housing 14a and either side of the slot 18, a row of raised projections 20 accommodate two small cylindrical spigots 21 projecting from either side of the carriage lever (Figure 7 and 10). The two rows of raised projections 20, immediately adjacent to the edge of the slot and along the entire length of the slot, present a cylindrical contour 22 with minimal clearance to the outer diameter of the carriage tube 13. When the carriage tube is assembled inside the housing it has a single degree of freedom, able to move linearly i.e. extend and retract. The position of the spigots 21 on the carriage lever 17, relative to the central axis of the needle (and carriage tube), creates a cantilever whose bending moment is a maximum where the carriage lever extends from the carriage tube 23. The opposing force resisting this bending moment ensures contact between the spigots and each row of raised projections; in a manner similar to a rack and pinion gear. This functionality enables the slider & needle to be selectively temporarily locked in place at any desired position along the length of the feature such that it allows for an inherently stable deployment of the needle in a partially retracted position or partially/fully extended position when the thumb grip is not depressed. Attached to the posterior of the lever, external to the housing, is the thumb grip 24. Pressing the thumb grip disengages the spigots. Held within the palm of the user's hand and with moderate pressure applied to the thumb grip simultaneous with distally biased pressure, the needle extends from the housing; the heel 25 of the lever compressing and clamping the flexible tube to move in unison with the needle. With sufficient extension of the needle, the cannula housing can then be manipulated ready for use together with the cord clamp. The plurality of ridges on the cord clamp are included so as to enable varying clamping force

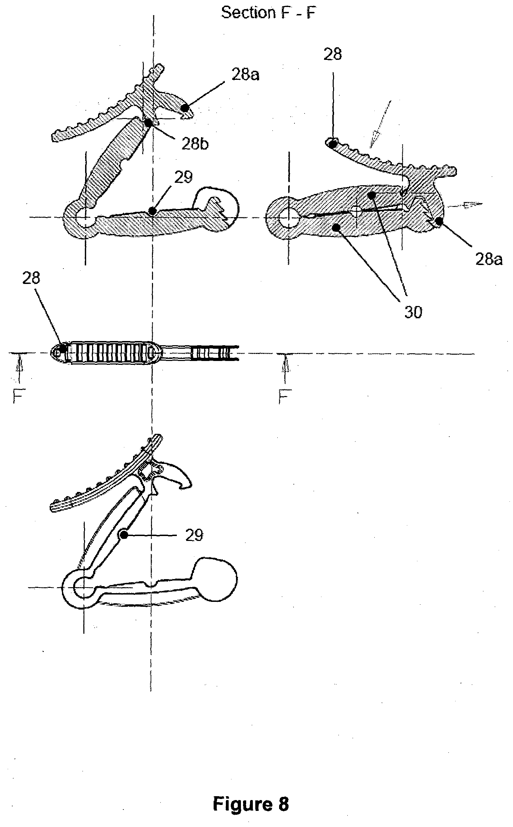

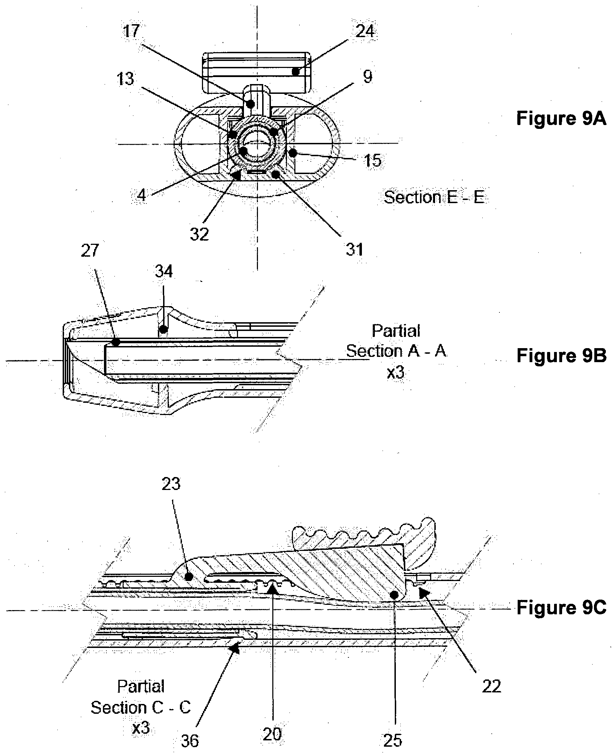

The cord clamp 26 is placed over the umbilical cord distal to the desired point of cannulation. It is intended to provide temporary occlusion of the umbilical vein, reducing the potential for blood loss, at that moment when the needle taper point 10 perforates the umbilical vein. The spherical tip 5 of the flexible cannula tubing, having a locally increased outside diameter, creates an annular seal 27 against the inside diameter of the needle (Figure 9); preventing leakage of cord blood past the spherical tip, where it may collect between the outside diameter of the flexible cannula tube and the inside diameter of the needle. Using the quick release lever 28 (Figure 8), the hook 28a rotates locally around a reduced cross section area 28b allowing the cord clamp to be removed and repositioned so that the cylindrical aperture 29 in the clamp jaws 30 is aligned with and allowed to clamp the umbilical cord over the needle 9.

Thereafter, the user can grip the cannula housing in one hand whilst guiding the proximal flexible cannula tube 30 through the cannula housing 14 with the other hand; the spherical tip 5 smoothly negotiating the inside of the umbilical vein.

With the flexible cannula sufficiently extended into the umbilical vein, the cord clamp is once again released and then repositioned so that the cylindrical aperture 29 in the clamp jaws 30 is aligned with and allowed to clamp the umbilical cord over the flexible cannula tube 4; the diameter of the cylindrical aperture being sufficiently large to prevent occlusion. Compressing the thumb grip 24 and applying a proximally biased pressure retracts the needle back to the initial position whereupon tactile feedback to the user will suggest resistance to any further retraction. Two (2) spherical protrusions 31 on the inside of the lower housing, posterior to the carriage tube 13, engage with the leading edge of the carriage tube. If the cannula is being used for in-utero collection and immediately thereafter, ex-utero collection, additional proximally biased pressure retracts the needle fully. However, if used for ex-utero this partial retraction creates a needle safe environment for assessing cord blood collection prior to a deliberate re-extension and re-cannulation.

In the partial retraction position, the two (2) spherical protrusions 31 on the lower housing, engage with the leading edge of the carriage tube. By applying sufficient proximal biased pressure to the thumb grip, the leading edge of the carriage will rise over the spherical projections; which offer a minimal contact area of interference 32 to the carriage tube. With continued proximally biased pressure all resistance is overcome and the needle will retract until the carriage lever stops against the proximal extremity of the slot 18.

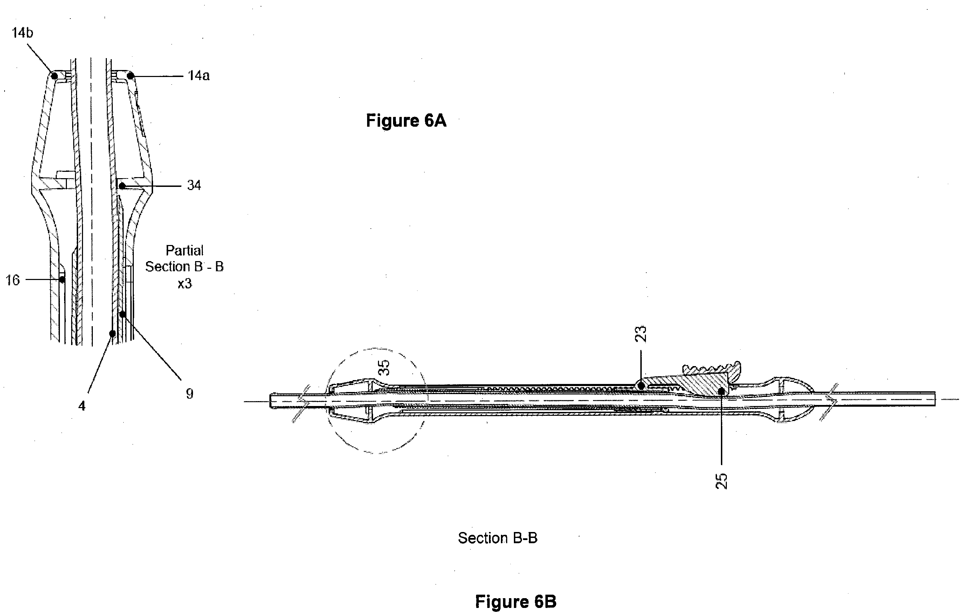

There are two methods of preventing the needle from being extended for re-use. Firstly, two (2) spherical depressions 33 in the carriage tube 13 will align with the aforementioned spherical projections 31 in the lower housing 14b. The position of alignment will coincide with the taper point of the needle coming to rest proximal to a longitudinal rib 34 in the upper housing (Figure 6). Due to the interference of these spherical protrusions against the carriage tube, combined with resistance to the compression of the carriage lever creating a moment around the contact point 32 of the spherical protrusions, the distal taper point now assumes an angular deflection 35 relative to the central axis of the needle i.e. when the needle was extended or partially retracted. Any attempt to re-extend the needle will result in the needle taper point colliding with the proximal side of this rib 34. Secondly and further proximally, a rectangular protrusion 36 from the inside of the lower housing aligns with an anterior slot 37 in the carriage tube, configured parallel to the centre axis (Figure 5 and 10). Distally biased pressure applied to the carriage lever 17 will force the proximal end of this slot 37 against the rectangular protrusion 36, ensuring the carriage remains fully retracted. With the needle fully retracted and locked, the cannula housing can be slid over the cannula tube and taped to the umbilical cord as required. In-utero cord blood collection can proceed followed by ex-utero should it be necessary.

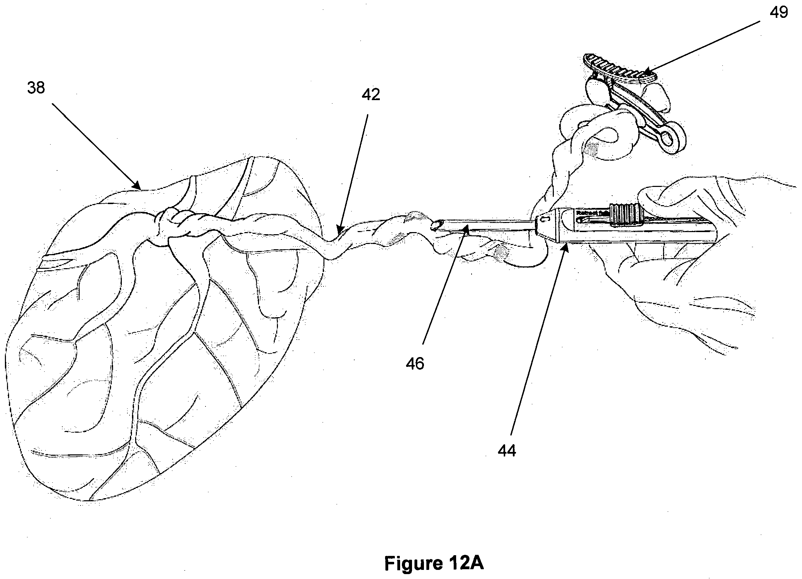

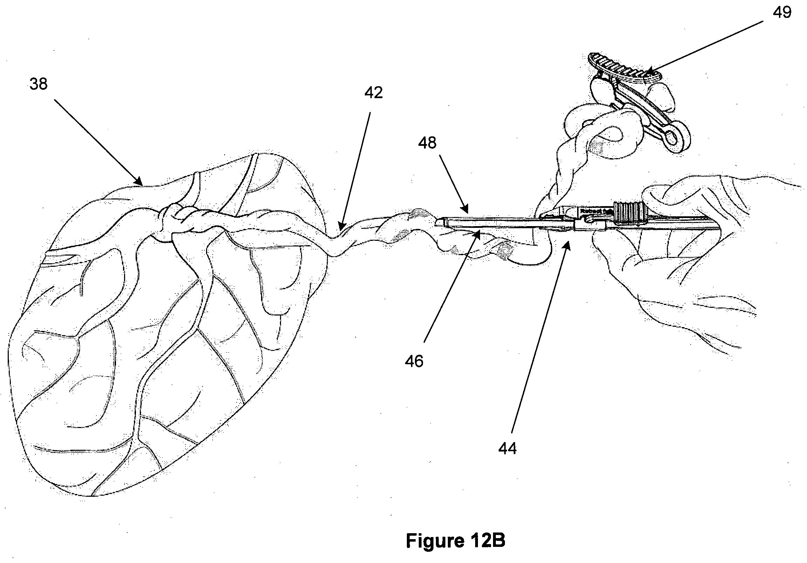

Figures 12A and 12B show the first step for the collection of cord blood. Here at placenta 38 has umbilical cord 42 attached from which the cord blood is to be harvested. A cannulation assembly 44 according to one embodiment of the present invention has the needle 46 extended outside the housing for penetration of the cord. The needle has a bore through which a tube 48 is placed. The tube 48 extends through the housing and through the bore of the needle 46 ready for insertion in the cord.

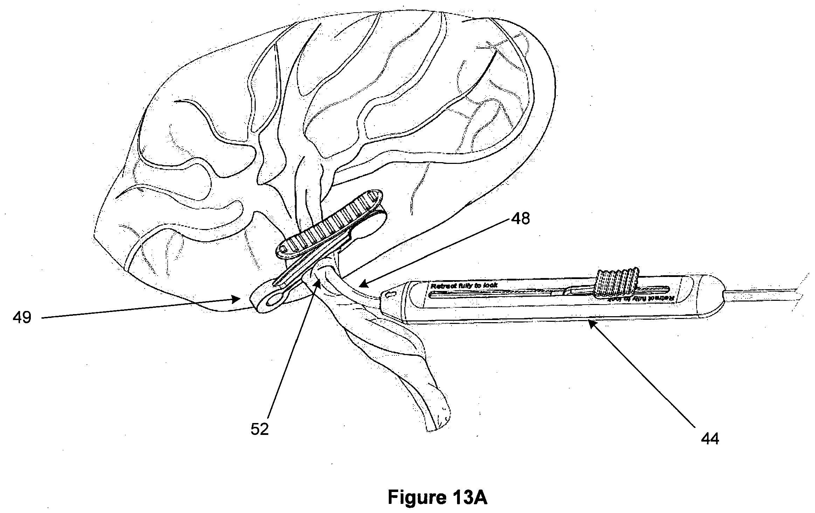

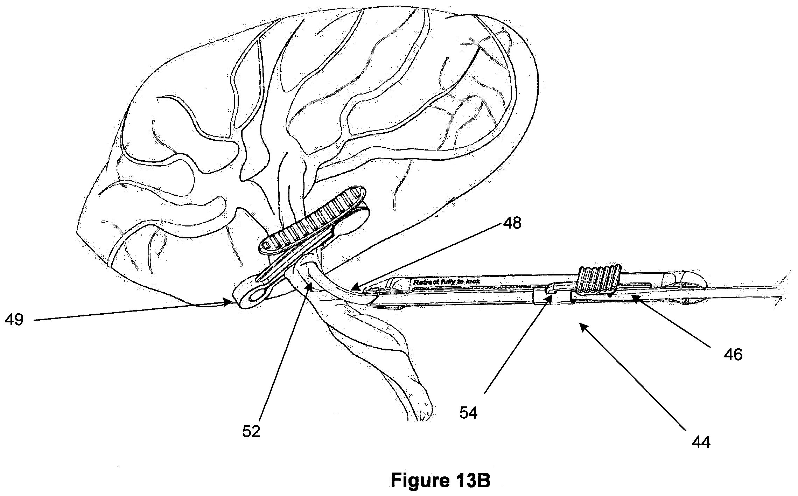

Figures 13A and 13B show the next step by which the needle having penetrated the cord 42 then allows the tube 48 to be inserted into the cord. The tube 48 is then pushed further into the cord so as to be well within the cord in order for harvesting. The needle is then retracted into the housing and locked in place so as to prevent further use of the cannulation assembly. This then leads to the image shown in Figures 13A and 13B whereby the needle has now been retracted. The tube 48 is inserted into the cord and the cord and indirectly the tube are then clamped by a clamp 49 at the cannulation site 52 so as to hold the tube and cord in place.

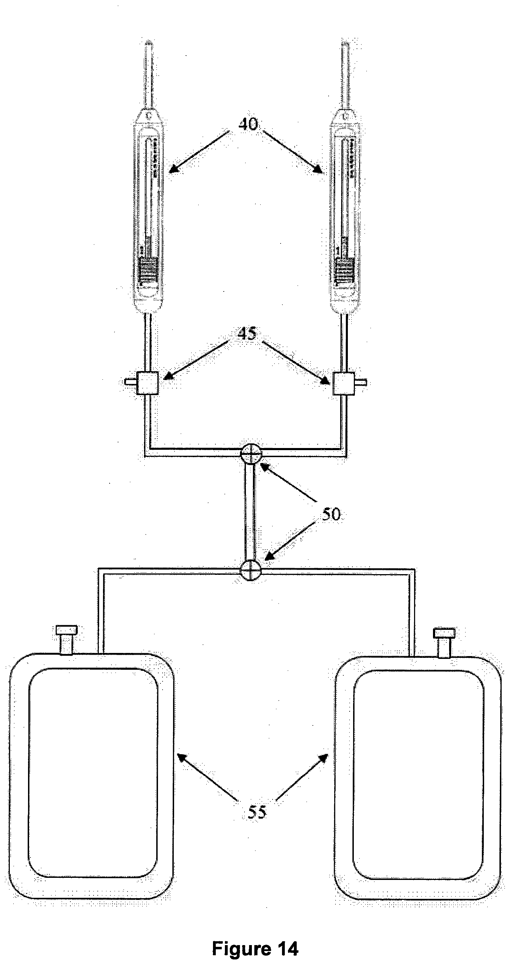

Figure 14 shows a possible arrangement of a full cannulation assembly kit. Here, a pair of cannulation assemblies 40 is connected by valves 45 and 50 to supply bags 55. It would be appreciated that in a full arrangement the cannulation assembly 40 will need to drain the harvested blood into a blood bag 55 with Figure 14 showing a full arrangement under optimal conditions. The dual arrangement shown in Figure 14 allows for multiple harvesting points and so accommodates multiple cannulation sites. Further, to ensure that no time is wasted in the critical few minutes from pre-delivery to post-delivery by having multiple blood bags 55 connected to the multiple cannulation assemblies, the volume of harvested blood can then be maximized. It would be appreciated that such an arrangement may have a single cannulation assembly, a single blood bag, or various combinations thereof. It would further be appreciated that multiple cannulation assemblies, that is, more than two may also be used. Further still, multiple blood bags 55 may also be attached in anticipation of a high supply of cord blood harvested.

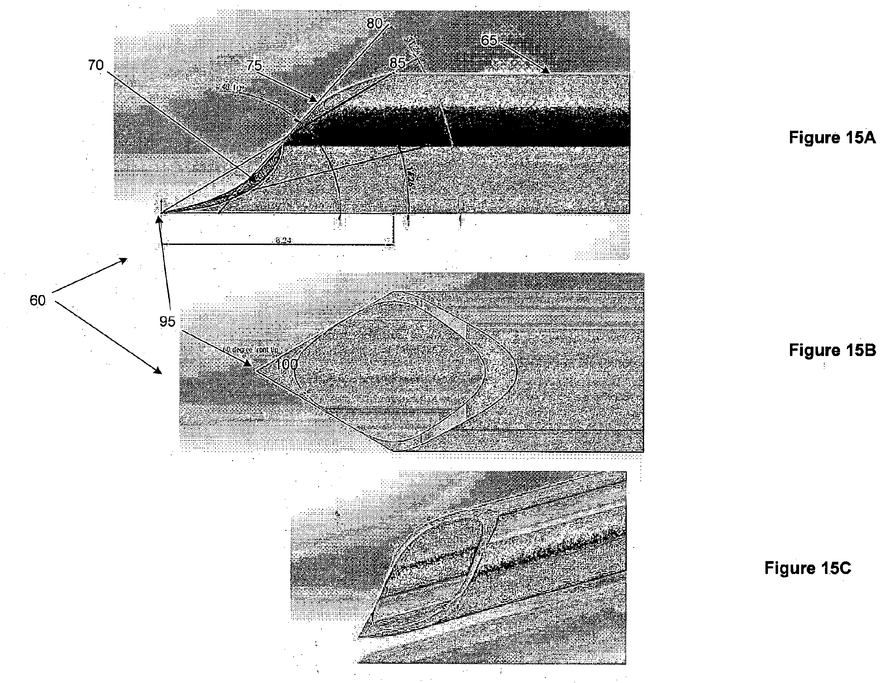

Figure 15A to 15C shows a side profile of a needle 65 for a cannula according to one embodiment of the present invention. In this embodiment, the bevel 60 of the needle is a reverse curve having a greater steepness 80 at the middle section of a spline profile. In the present embodiment, the reverse curve is a "S" spline shaped needle bevel design which is designed with a smooth and continuously varying bevel gradient 70, 75.

The primary advantage of the proposed design is the shallow proximal tip and reduced bevel length. The advantage of a shallow 90 and narrow proximal needle tip 95 is that it will greatly reduce the initial penetration force required. The reduced bevel length helps to prevent puncturing through the cord as it enables the needle bevel to be fully enclosed by the umbilical cord with minimal depth of insertion of the needle.

The proximal tip 95 of the needle has a shallow gradient 90 (drawn as approximately 14 degrees in Figure 15 for the purposes of a non-limiting example). The mid-section of the bevel has a much steeper gradient 80 (drawn as 48 degrees as a non-limiting example). The final section of the needle bevel resumes to a shallow gradient. The proximal tip 95 is also suitably narrowed (drawn as 60 degree V shaped wedge 100 for the purposes of a non-limiting example).

The steep mid-section creates a tangible increased resistance to insertion such that the operator has a haptic "notch" to indicate that the needle is mid-way into the cord. This will help the operator to differentiate the depth of insertion, and prevent the operator from puncturing right through the cord

## Claims

1. A cannulation assembly comprising
a housing (14a, 14b);
a needle (9) selectively extendable from a proximal end of said housing;
a tube (4) arranged to pass through the housing and through a bore of said needle such that an insertion end (5) of said tube is arranged to selectively project from an insertion end (10) of said needle;
a slide mounted to the needle in sliding engagement with the housing and arranged to move the needle from a retracted position to an insertion position such that the needle projects from said housing;
wherein said slide is further arranged to move the needle from the insertion position to a locked position such that the needle is fully retracted;
**characterised in that** the insertion end of the tube is spherical in shape, said spherical insertion end arranged to form an annular seal (27) against an inside diameter of the needle.

2. The cannulation assembly according to claim 1, wherein said housing includes a first lock arranged to irrevocably lock the needle inside the housing whilst in the locked position.

3. The cannulation assembly according to claim 2, wherein the first lock includes corresponding lugs on the slide and the housing, said lugs arranged to permit the slide to retract into the locked position but prevent the slide from moving from the locked position through an interference between the respective lugs.

4. The cannulation assembly according to any one of claims 1 to 3, wherein the needle is arranged to penetrate an umbilical cord (42), an external diameter of said needle corresponding to the bore of said umbilical cord.

5. The cannulation assembly according to any one of claims 1 to 4,wherein said insertion end of said tube is enlarged so as to seal an interstitial space between the needle bore and tube.

6. The cannulation assembly according to claim 2 or any of claims 3 to 5 when appended to claim 2, wherein said housing includes a second lock intermediate the locked position and the proximal position, said second lock arranged to selectively release the needle on engagement.

7. The cannulation assembly according to any one of claims 1 to 6, wherein a bevel of said needle includes a reverse curve.

8. The cannulation assembly according to claim 7, wherein the reverse curve is a "S" shaped spline.

## Patentansprüche

1. Punktionsanordnung, umfassend:
ein Gehäuse (14a, 14b);
eine Nadel (9), welche selektiv von einem proximalen Ende des Gehäuses ausfahrbar ist;
einen Schlauch (4), welcher angeordnet ist, um durch das Gehäuse und durch eine Bohrung der Nadel zu dringen, sodass ein Einführende (5) des Schlauchs angeordnet ist, um selektiv von einem Einführende (10) der Nadel vorzustehen;
einen Schlitten, der an der Nadel in gleitendem Eingriff mit dem Gehäuse montiert ist und angeordnet ist, um die Nadel von einer eingezogenen Position in eine Einführposition zu bewegen, sodass die Nadel vom Gehäuse vorsteht;
wobei der Schlitten ferner angeordnet ist, um die Nadel von der Einführposition in eine verriegelte Position so zu bewegen, dass die Nadel vollständig eingezogen ist;
**dadurch gekennzeichnet, dass** das Einführende des Schlauchs eine Kugelform aufweist, wobei das kugelförmige Einführende angeordnet ist, um eine Ringdichtung (27) gegenüber einem Innendurchmesser der Nadel zu bilden.

2. Punktionsanordnung nach Anspruch 1, wobei das Gehäuse eine erste Verriegelung umfasst, welche angeordnet ist, um die Nadel unwiederbringlich innerhalb des Gehäuses in der verriegelten Position zu verriegeln.

3. Punktionsanordnung nach Anspruch 2, wobei die erste Verriegelung entsprechende Laschen auf dem Schlitten und in dem Gehäuse umfasst, wobei die Laschen angeordnet sind, um das Einziehen des Schlittens in die verriegelte Position zu ermöglichen, während der Schlitten durch einen Eingriff zwischen den Laschen daran gehindert wird, sich von der verriegelten Position zu bewegen.

4. Punktionsanordnung nach einem der Ansprüche 1 bis 3, wobei die Nadel angeordnet ist, um in eine Nabelschnur (42) einzudringen, wobei ein Außendurchmesser der Nadel der Bohrung der Nabelschnur entspricht.

5. Punktionsanordnung nach einem der Ansprüche 1 bis 4, wobei das Einführende des Schlauchs ausgeweitet ist, um einen Zwischenraum zwischen der Nadelbohrung und dem Schlauch abzudichten.

6. Punktionsanordnung nach Anspruch 2 oder einem der Ansprüche 3 bis 5, wenn abhängig von Anspruch 2, wobei das Gehäuse eine zweite Verriegelung zwischen der verriegelten Position und der proximalen Position umfasst, wobei die zweite Verriegelung angeordnet ist, um die Nadel beim Eingriff selektiv zu lösen.

7. Punktionsanordnung nach einem der Ansprüche 1 bis 6, wobei eine Schräge der Nadel eine gegensinnige Kurve umfasst.

8. Punktionsanordnung nach Anspruch 7, wobei die gegensinnige Kurve ein S-förmiger Spline ist.

## Revendications

1. Ensemble de canulation, comprenant :
un logement (14a, 14b) ;
une aiguille (9) pouvant s'étendre sélectivement à partir d'une extrémité proximale dudit logement ;
un tube (4) configuré pour passer à travers le logement et à travers un alésage de ladite aiguille, de sorte qu'une extrémité d'insertion (5) dudit tube est configurée pour déborder sélectivement à partir d'une extrémité d'insertion (10) de ladite aiguille ;
un coulisseau monté sur l'aiguille, engagé de manière coulissante dans le logement et configuré pour déplacer l'aiguille d'une position rétractée vers une position d'insertion, l'aiguille débordant ainsi à partir dudit logement ;
dans lequel ledit coulisseau est en outre configuré pour déplacer l'aiguille de la position d'insertion vers une position verrouillée, de sorte que l'aiguille est entièrement rétractée ;
**caractérisé en ce que** l'extrémité d'insertion du tube a une forme sphérique, ladite extrémité d'insertion étant configurée pour former un joint d'étanchéité annulaire (27) par rapport à un diamètre intérieur de l'aiguille.

2. Ensemble de canulation selon la revendication 1, dans lequel ledit logement inclut un premier verrou configuré pour verrouiller de manière irrévocable l'aiguille à l'intérieur du logement pendant qu'elle se trouve dans la position verrouillée.

3. Ensemble de canulation selon la revendication 2, dans lequel le premier verrou inclut des pattes correspondantes sur le coulisseau et le logement, lesdites pattes étant configurées pour permettre la rétraction du coulisseau dans la position verrouillée, tout en empêchant le déplacement du coulisseau de la position verrouillée par suite d'une interférence entre les pattes respectives.

4. Ensemble de canulation selon l'une quelconque des revendications 1 à 3, dans lequel l'aiguille est configurée pour pénétrer dans un cordon ombilical (42), un diamètre extérieur de ladite aiguille correspondant à l'alésage dudit cordon ombilical.

5. Ensemble de canulation selon l'une quelconque des revendications 1 à 4, dans lequel ladite extrémité d'insertion dudit tube est élargie, de sorte à établir l'étanchéité d'un espace interstitiel entre l'alésage de l'aguille et le tube.

6. Ensemble de canulation selon la revendication 2 ou selon l'une quelconque des revendications 3 à 5 dépendant de la revendication 2, dans lequel ledit logement inclut un deuxième verrou intermédiaire entre la position verrouillée et la position proximale, ledit deuxième verrou étant configuré pour libérer sélectivement l'aiguille lors de son engagement.

7. Ensemble de canulation selon l'une quelconque des revendications 1 à 6, dans lequel un biseau de ladite aiguille inclut une courbe inversée.

8. Ensemble de canulation selon la revendication 7, dans lequel la courbe inversée est une cannelure en forme de S.
